Europäisches Patentamt

(19) **European Patent Office**

Office européen des brevets

(11) Publication number: **0 325 348**
**A2**

(12) # EUROPEAN PATENT APPLICATION

(21) Application number: **89300140.4**

(51) Int. Cl.⁴: **C12N 9/00 , C12N 9/80**

(22) Date of filing: **09.01.89**

---

The microorganism(s) has (have) been deposited with National Collections of Industrial and Marine Bacteria under number NCIB 10515.

(30) Priority: **21.01.88 GB 8801303**

(43) Date of publication of application:
**26.07.89 Bulletin 89/30**

(84) Designated Contracting States:
**AT BE CH DE ES FR GB IT LI NL SE**

(71) Applicant: **IMPERIAL CHEMICAL INDUSTRIES PLC**
**Imperial Chemical House Millbank**
**London SW1P 3JF(GB)**

(72) Inventor: **Carver, Mark Andrew**
**Hallswood 503 Yarm Road**
**Eaglescliffe Stockton-on-Tees**
**Cleveland(GB)**

(74) Representative: **Aufflick, James Neil et al**
**Imperial Chemical Industries PLC Legal**
**Department: Patents PO Box 6 Bessemer**
**Road**
**Welwyn Garden City Herts, AL7 1HD(GB)**

---

(54) **Improved method for separating soluble enzymes from cell débris.**

(57) A method for separating a water soluble enzyme from undissolved material and/or non-enzyme protein (particularly cell débris) present in an aqueous medium containing the enzyme in which the aqueous medium is contacted with a clay and is thereafter subjected to a separation treatment. Preferred clays are smectite clay such as bentonites which are suitably added to the medium in water-swollen form.

EP 0 325 348 A2

## Improved method for separating soluble enzymes from cell débris

This invention relates to a method for separating water soluble enzymes from undissolved material and/or non-enzyme proteins present in aqueous media containing the enzymes in solution.

In order to obtain intracellular enzymes from cells containing them, the cells are broken either by mechanical means or by some other treatment for instance by heating or by treatment with acidic or basic reagents, detergents or enzymes e.g. lysozyme. Combinations of such methods may also be used. The first product of cell breakdown is an aqueous suspension containing broken cells, cell fragments and other cell débris with water soluble enzymes and possible other proteinaceous material in solution. Separation of the solution containing the soluble enzymes from broken cells and other débris can be carried out by centrifugation. However this gives solutions which still contain discernable amounts of débris unless very powerful centrifuges capable of attaining high G forces are used in the separation. The presence of such discernable amounts of débris may interfere with the further purification and/or use of the soluble enzymes. Alternatively the solutions containing the soluble enzymes can be separated from the broken cells and other undissolved débris by treatment of the suspensions containing them with polymeric flocculants. However such treatments also give rise to problems due to discernable amounts of the flocculants remaining associated with the enzyme-containing solutions which is not desirable.

According to the present invention we provide a method for separating a water soluble enzyme from undissolved material and/or non-enzyme protein present in an aqueous medium containing the enzyme in solution wherein the aqueous medium is contacted with a clay and thereafter is subjected to a treatment to separate undissolved material and/or non-enzyme protein from the aqueous medium.

The method of the invention can be used in particular to separate water soluble enzymes from cell débris and other undissolved material obtained when cells containing the enzymes are broken down. A wide variety of clays and a wide range of conditions may be used in performing the method. However the optimum clay and the optimum conditions vary depending upon the water soluble enzyme which is to be separated. For many water soluble enzymes smectite clays such as bentonites are very suitable. Bentonites swell when water is added to them and, when used in the method of the invention, are preferably added to the medium from which a water soluble enzyme is to be separated in water-swollen form. Particularly suitable bentonites are those obtained from the well-known deposits in Wyoming, USA for example "ORGANOSORB" (Registered Trade Mark) Wyoming bentonite.

Any water soluble enzyme may be separated from solid material suspended in aqueous media using the method of the invention although the optimum clay and conditions to be used vary depending upon the enzyme. Amidase is a water soluble enzyme which may very usefully be separated using the method. In our European Published Patent Applicati on No. 272025 we describe an amidase induced in cells of Methylophilus-methylotrophus AS-1 (a culture of which is deposited at and available as strain NCIB 10515 from the National Collections of Industrial and Marine Bacteria (NCIMB), PO Box 31, 135 Abbey Road, Aberdeen, AB9 8DG, Scotland, UK). This amidase is very readily separated from broken cells of strain AS-1 using the method of the invention.

In the method of the invention clay is suitably added to the aqueous medium containing the enzyme to be separated. Suitably sufficient clay is added to adsorb the cell débris and other undissolved material without absorbing significant amounts of the enzyme to be separated. In any particular instance if an optimum clay is added in an optimum amount, dissolved non-required proteins in addition to undissolved material can be absorbed onto the clay and thereby separated from the required water soluble enzyme. In many instances the amount of clay added to the aqueous medium is suitably in the range 0.1 to 2 parts clay to 10 parts suspended solids in the medium. A particularly suitable proportion is 1 part clay to 10 parts suspended solids. When a water swollen thixotropic clay is used these proportions refer to clay solids. Optimum values for other conditions during clay addition vary depending upon the enzyme to be separated and the clay to be used. Very often pH values near neutral are suitable, e.g. in the range pH 6 to 8 and particularly in the range 6 to 7. Suitable temperatures are often in the range 10° to 60°C.

After treatment of the aqueous medium with clay, solids may be separated from the aqueous medium containing the required water soluble enzyme by any suitable means, e.g. by centrifugation.

The invention is illustrated by the following Example:-

EXAMPLE

A cell slurry of Methylophilus methylotrophus strain NCIB 10515 comprising 10% dry solids was broken by french pressing in the presence of DNA ase. The material produced was divided into 3 parts. One part served as a control and was not treated with a clay slurry. To the other parts "ORGANOSORB" bentonite clay was added in amounts respectively of 10% and 20% $^w$/w (weight dry clay per weight dry cell solids) - recorded parameters have been corrected for dilution due to the clay slurry. All 3 parts were then subjected to a 1 minute centrifugation at 3000 G in a "SORVAL" RC5B centrifuge, rotor type SM24. The supernatant liquid was decanted and was assayed by standard methods for dry solids content, protein concentration, amidase activity, glucose-6-phosphate dehydrogenase (G6PD) activity and soluble cytochrome C content.

The results are set out in the Table. They show that the percentage solids and total protein concentration in the clay treated parts was considerably less than in the control. Amidase activity was not significantly reduced in either of the clay treated parts showing that this was an optimum treatment for this enzyme. However cytochrome C activity was significantly reduced in both clay treated parts and glucose-6-phosphate-dehydrogenase activity was significantly reduced when the 20% clay treatment was used. This shows that for these enzymes, whilst the solids and other proteins separation was successful, this was achieved at the expense of some loss of enzyme activity and in these cases further optimisation would be desirable.

## Table

|  | % solids $^w$/v | total protein concentration | Amidase activity (% control) | G6PD activity (% control) | cytochrome C activity (% control) |
|---|---|---|---|---|---|
| Control | 1.54 | 32.8 | 100 | 100 | 100 |
| 10% clay treated | 0.43 | 13.1 | 98.4 | 101.2 | 69.2 |
| 20% clay treated | 0.45 | 9.4 | 95.2 | 64 | 46.8 |

## Claims

1. A method for separating a water soluble enzyme from undissolved material and/or non-enzyme protein present in an aqueous medium containing the enzyme in solution wherein the aqueous medium is contacted with a clay and thereafter is subjected to a treatment to separate undissolved material and/or non-enzyme protein from the aqueous medium.

2. A method according to claim 1 wherein the clay is a smectite clay.

3. A method according to claim 2 wherein the clay is a bentonite.

4. A method according to claim 3 wherein the clay is added in water-swollen form to the aqueous medium containing the enzyme.

5. A method according to any one of the preceding claims wherein the water soluble enzyme is an amidase.

6. A method according to claim 5 wherin the amidase is derived from a strain of Methylophilus methylotrophus.

7. A method according to any one of the preceding claims wherein the clay is added to the aqueous medium containing the water soluble enzyme to be separated.

8. A method according to any one of the preceding claims wherein the aqueous medium is contacted with sufficient clay to absorb any undissolved material present without absorbing significant amounts of the water soluble enzyme to be separated.

9. A method according to any one of the preceding claims wherein the aqueous medium is contacted with a clay in an amount in the range 0.1 to 2 parts clay to 10 parts suspended solids in the medium, these proportions referring to clay solids when a water swollen clay is used.

10. A method according to any one of the preceding claims wherin, after treatment of the aqueous medium with clay, undissolved material and/or non-enzyme protein is separated from the medium by centrifugation.